# EUROPEAN PATENT APPLICATION

(11) **EP 4 194 032 A1**
(43) Date of publication of application: **14.06.2023**
(21) Application number: 21852356.1
(22) Date of filing: 02.08.2021
(51) Int. Cl.: A61M 5/24, A61M 5/46

(54) **MEDICAL DOSING DEVICE**

(30) Priority: 05.08.2020 JP 2020132916
(71) Applicant: Lightnix, Inc., Nishinomiya-shi, Hyogo 662-0812 (JP)
(72) Inventor: FUKUDA, Moe, Soka-shi, Saitama 340-0015 (JP); KOBAYASHI, Noriyuki, Soka-shi, Saitama 340-0015 (JP)
(74) Representative: Ström & Gulliksson AB
(86) International application number: PCT/JP2021/028652
(87) International publication number: WO 2022/030457

(57) **Abstract**

The present invention comprises: a needle 2; a cartridge 3 which is capable of storing therein a liquid medicine LM to be administered into a living body IN by means of the needle 2; a case 4 which has an open or openable first end 45 to be pressed against the skin SK, and in which the needle 2 and the cartridge 3 are stored in such a manner as to be movable toward the first end 45 side, the needle 2 being capable of moving to the outside of the first end 45 in response to the movement of the needle 2 toward the first end 45 side; and a pressing force control member 6 which intervenes so as to generate a reactive force between the cartridge 3 and the case 4 and which controls a pressing force exerted on the skin SK by the case 4 while the needle 2 moves toward the first end 45 side. The present invention is configured such that: in the process of piercing by the needle 2, a reactive force is continuously generated by the pressing force control member 6 and the pressing force exerted on the skin SK by the case 4 is controlled at a substantially constant level, whereby the piercing depth by the needle 2 is kept substantially constant at the time of completion of the piercing by the needle 2; and in the process of discharging the liquid medicine LM stored in the cartridge 3 through the needle 2 for administration, the reactive force by the pressing force control member 6 is generated continuously, whereby the piercing depth by the needle 2 is kept substantially constant.

## Description

### TECHNICAL FIELD

The present invention relates to a liquid medicine administration device.

### BACKGROUND ART

Conventionally, intradermal administration devices have been developed for the purpose of decreasing the amount of administration of liquid medicine. The development is not easy because there are many design matters to be satisfied. The design matters to be satisfied include, for example, to control the insertion depth of a needle to be intradermally inserted in order to accurately administer liquid medicine, to prevent an accident of inserting a used needle and to disable repeated use.

Specifically, each of Patent Documents 1 and 2 discloses a device with a cylindrical or dome-shaped structure that is additionally provided at a location near the needle point, and maintains the thickness of a skin constant by the structure stretching out a skin when coming into contact with the skin.
Patent Document 1: PCT International Publication No. WO2013/046867
Patent Document 2: PCT International Publication No. WO2013/046868

### DISCLOSURE OF THE INVENTION

### Problems to be Solved by the Invention

The device provided with the cylindrical or dome-shaped structure near the needle point, makes it possible to stabilize the thickness of a skin, but is unable to stabilize the insertion depth of a needle due to differences in a pressing force applied by an operator such as a nurse. Therefore, it is not easy to cause the insertion depth of a needle that is intradermally inserted to be constant.

Such a problem is not limited to the case of an intradermal administration device but can exist in common in the cases of devices for administration into subcutaneous tissue, muscle or other living bodies.

The present invention has been made in view of the above disadvantages, and an object is to provide an administration device capable of easily causing the insertion depth of a needle to be substantially constant.

### Means for Solving the Problems

(1) The present invention is directed to an administration device including: a needle to be inserted into a living body; a cartridge capable of accommodating liquid medicine to be administered into the living body through the needle; a case having an open or openable first end to be pressed against a skin, and housing therein the needle and the cartridge in such a manner that the needle and the cartridge are movable toward the first end side, the needle being capable of advancing to an outside of the first end by being moved toward the first end side; and a pressing force control member intervening between the cartridge and the case so as to generate a reaction force and controlling a pressing force of the case against the skin when the needle moves toward the first end side. The pressing force control member is capable of, in a process of discharging and administering the liquid medicine in the cartridge through the needle into the living body, continuing generating the reaction force.
   Further, the present invention is directed to an administration device including: a needle to be inserted into a living body; a cartridge capable of accommodating liquid medicine to be administered into the living body through the needle; a case having an open or openable first end to be pressed against a skin, and housing therein the needle and the cartridge in such a manner that the needle and the cartridge are movable toward the first end side, the needle being capable of advancing to the outside of the first end by being moved toward the first end side; and a pressing force control member intervening between the cartridge and the case so as to generate a reaction force and controlling a pressing force of the case against the skin when the needle moves toward the first end side. The administration device is configured to insert the needle into the living body by, in a state in which the first end of the case is pressed onto the skin, causing the needle to move through an opening of the first end so as to advance the needle to the outside of the first end. In a process of inserting the needle into the living body by causing the needle to move through the opening of the first end so as to advance the needle to the outside of the first end, the administration device causes the pressing force control member to continue generating the reaction force and controls the pressing force of the case against the skin to be substantially constant so that insertion depth of the needle is substantially constant in a state in which insertion of the needle into the living body is completed, and in a process of discharging and administering the liquid medicine in the cartridge through the needle into the living body, the administration device causes the pressing force control member to continue generating the reaction force so that the insertion depth of the needle is substantially constant.
(2) In response to the cartridge being released from a force applied toward the first end side, the pressing force control member may cause the cartridge to move integrally with the needle toward a side opposite to the first end relative to the case.
(3) In a state in which movement of the cartridge integrated with the needle is prevented after completion of the administration of the liquid medicine, the pressing force control member may cause the cartridge to move integrally with the needle toward the side opposite to the first end side relative to the case.
(4) There may be provided a notification mechanism configured to be engaged at a time of the completion of the administration of the liquid medicine to thereby provide notification of the completion of the administration of the liquid medicine to the operator.
(5) There may be provided a needle base supporting the needle at a position away from the cartridge and being in a state of fixation to the case in an initial state, and becoming movable integrally with the needle toward the first end side upon being released from the fixation; and a fixation release mechanism configured to release the fixation after the cartridge that has moved toward the first end side is integrated with the needle.
(6) There may be provided a pushing member configured to apply a force toward the first end side to the cartridge by moving toward the first end side by being pushed by an operator, and the cartridge may include: a cartridge body accommodating the liquid medicine; and a piston configured to push out the liquid medicine through the needle by being pushed by the pushing member and moving toward the first end side relative to the cartridge body.
(7) The present invention is directed to the administration device for use for intradermal administration.

### Effects of the Invention

According to the present invention, it is possible to provide an administration device capable of easily causing the insertion depth of a needle to be substantially constant.

### BRIEF DESCRIPTION OF THE DRAWINGS

FIG. 1 is an external perspective view of an administration device according to an embodiment of the present invention;
FIG. 2 is an exploded perspective view of the administration device shown in FIG. 1;
FIG. 3 is an exploded sectional perspective view of the administration device shown in FIG. 1;
FIG. 4A is a sectional perspective view of the administration device shown in a direction of an arrow X-X in FIG. 1 and shows an initial state;
FIG. 4B is a sectional perspective view of the administration device shown in the direction of the arrow X-X in FIG. 1 and shows an engaged state in which a cartridge is integrated with a needle;
FIG. 5A is a sectional view of the administration device shown in the direction of the arrow X-X in FIG. 1 and shows the initial state (a diagram corresponding to FIG. 4A);
FIG. 5B is a sectional view of the administration device shown in the direction of the arrow X-X in FIG. 1 and shows the engaged state in which the cartridge is integrated with the needle (a diagram corresponding to FIG. 4B);
FIG. 5C is a sectional view of the administration device shown in the direction of the arrow X-X in FIG. 1 and shows a state at the time when administration of liquid medicine is started;
FIG. 5D is a sectional view of the administration device shown in the direction of the arrow X-X in FIG. 1 and shows a state at the time when the administration of the liquid medicine is completed; and
FIG. 5E is a sectional view of the administration device shown in the direction of the arrow X-X in FIG. 1 and shows a state in which the needle is housed in a lower case after administration of the liquid medicine is completed.

### PREFERRED MODE FOR CARRYING OUT THE INVENTION

An embodiment according to the present invention will be described below in detail with reference to drawings. In each drawing, an arrow F indicates a forward direction; an arrow B indicates a backward direction; an arrow L indicates a left direction; an arrow R indicates a right direction; an arrow U indicates an upward direction; and an arrow D indicates a downward direction. Though, description will be made using the directions in the present specification for convenience, a posture at the time of using an administration device 1 is not limited to a particular posture. That is, though the administration device 1 is described as being used, being pointed in the downward direction D, the administration device 1 can be used being pointed in the upper direction U. The downward D corresponds to a direction toward the "first end side" in claim 1.

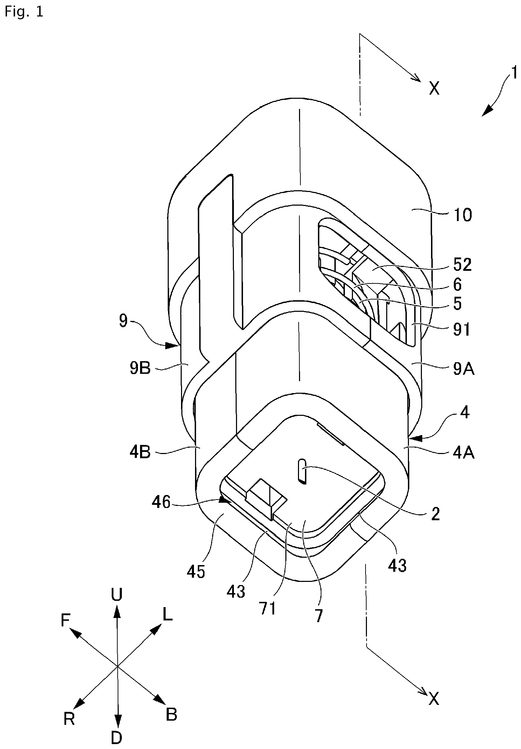

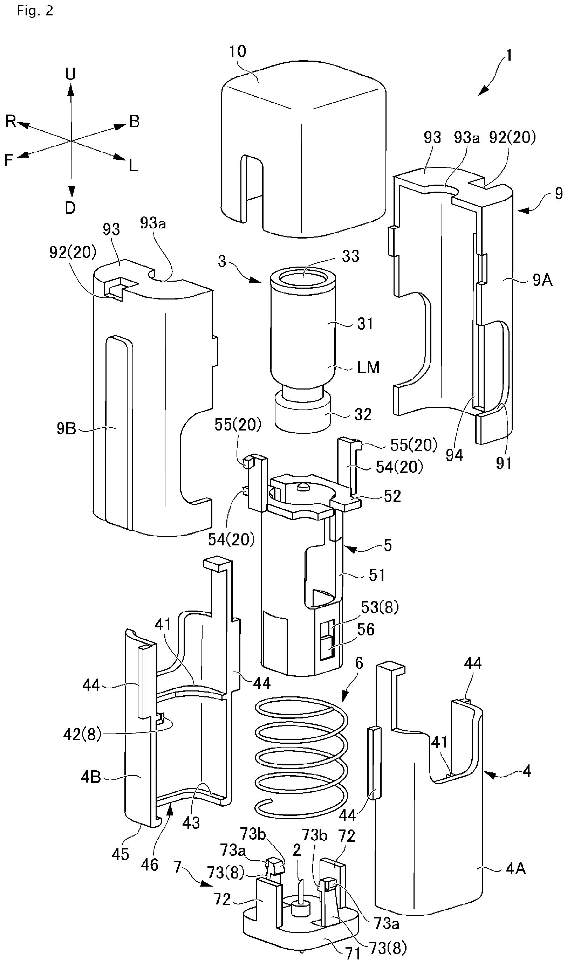

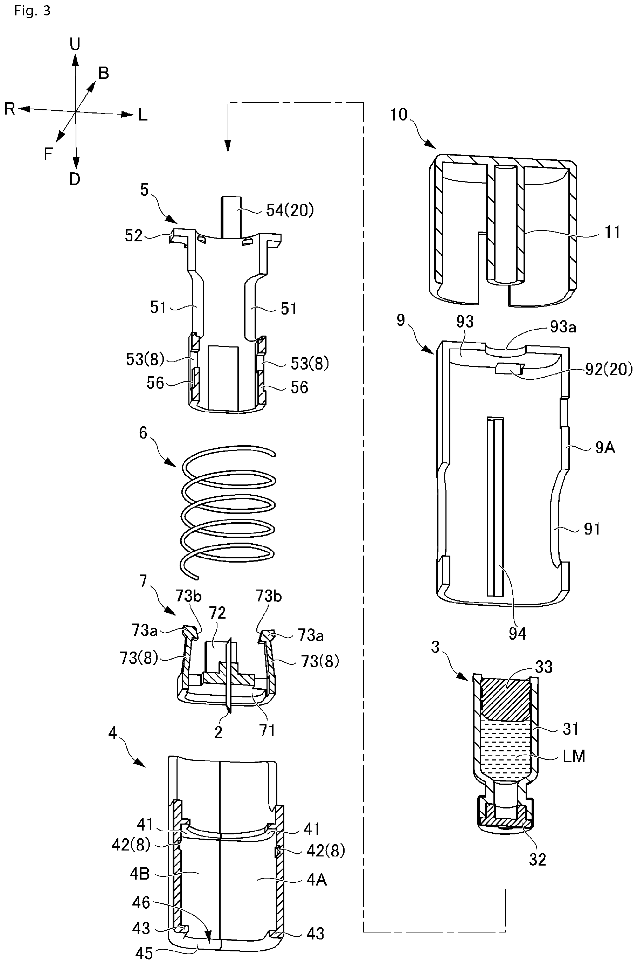

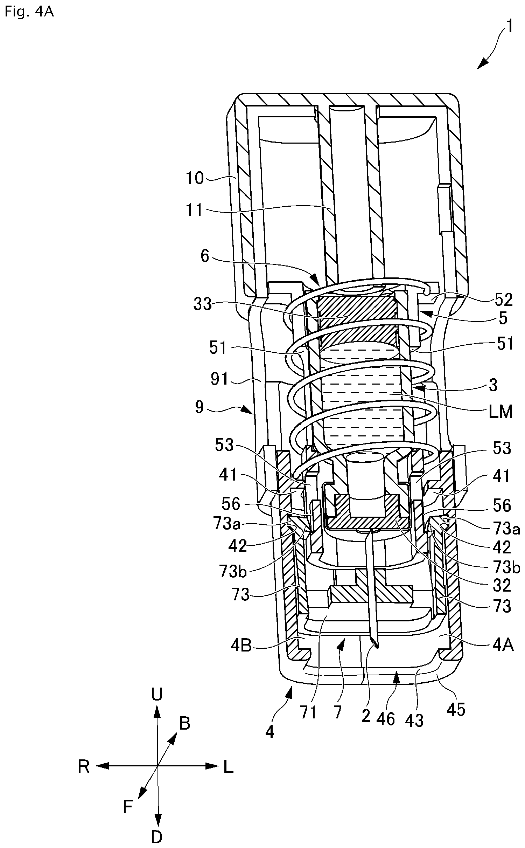

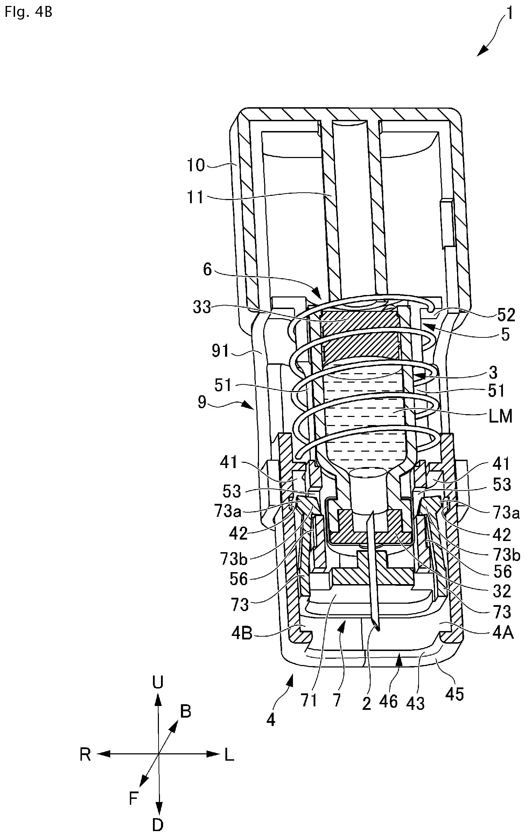

First, a configuration of the administration device 1 will be described, using FIGS. 1 to 4B. FIG. 1 is an external perspective view of an administration device according to an embodiment of the present invention. FIG. 2 is an exploded perspective view of the administration device shown in FIG. 1. FIG. 3 is an exploded sectional perspective view of the administration device shown in FIG. 1. FIG. 4A is a sectional perspective view of the administration device shown in a direction of an arrow X-X in FIG. 1 and shows an initial state. FIG. 4B is a sectional perspective view of the administration device shown in the direction of the arrow X-X in FIG. 1 and shows an engaged state in which a cartridge is integrated with a needle.

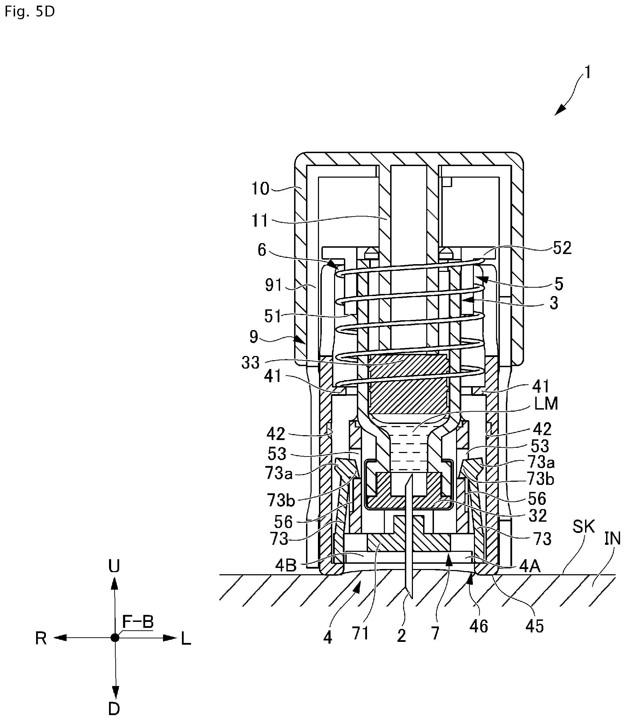

The administration device 1 shown in FIGS. 1 to 4B is an intradermal syringe for administrating liquid medicine LM between the epidermis and the dermis, that is, into the intradermal IN (see FIG. 5D and the like). Specifically, the administration device 1 includes a needle 2, a cartridge 3, a lower case 4 (case), a middle case 5, a spring 6 (a pressing force control member), a needle base 7, a fixation release mechanism 8, an upper case 9, a head cover 10, a notification mechanism 20 and the like.

The needle 2 is arranged to extend along the upper direction U and the lower direction D (a vertical direction). After the upward direction U side of the needle 2 breaks a seal 32 of the cartridge 3 by piercing the seal 32, the downward direction D side of the needle 2 is inserted into the intradermal IN (see FIG. 5D and the like). Thereby, the needle 2 becomes a flow path for the liquid medicine LM when the liquid medicine LM accommodated in the cartridge 3 is administered into the intradermal IN. The needle 2 is housed in the lower case 4 except at the time of being inserted and is, in the initial state, fixed to a position away from the cartridge 3 by being supported by the needle base 7. In response to the fixation being released, the needle 2 becomes movable integrally with the needle base 7 in the downward direction D.

The material of the needle 2 may be a metal material or may be resin having a lower stiffness and a higher flexibility in comparison with metal materials.

The cartridge 3 is a prefilled-type cartridge in which the liquid medicine LM to be administered into the intradermal IN through the needle 2 is accommodated. The cartridge 3 seals and stores the liquid medicine LM to prevent deterioration. The cartridge 3 has a cartridge body 31, the seal 32 and a piston 33. By the needle 2 piercing the seal 32 at the time of use, use of the liquid medicine LM is enabled. The cartridge 3 is housed in the middle case 5. The cartridge 3 and the middle case 5 are integrated with each other. The cartridge 3 is housed in the lower case 4 being integrated with the middle case 5 in such a manner as to be maintained at a predetermined position by the spring 6 in a coil shape intervening between the lower case 4 and the middle case 5. By a force in the downward direction D being applied to a pushing member 11 of the head cover 10, the cartridge 3 moves in the downward direction D being integrated with the middle case 5, against a reaction force of the spring 6. In response to the application of the force being released, the cartridge 3 moves in the upward direction U being integrated with the middle case 5, accompanying stretching of the spring 6 (that is, by the reaction force of the spring 6).

The cartridge body 31 is such a cylinder that both ends in the upward direction U and the downward direction D are open, and may be configured with a transparent material though the material is not especially limited. Here, "transparent" may be "transparent" or "translucent". Specifically, anything that is highly transparent is possible, and such that is less transparent than glass like a plastic material (transparent polypropylene or the like) is also possible. The cartridge body 31 may be a colored transparent container (for example, a colored vial bottle) to prevent deterioration of the accommodated liquid medicine LM. The cartridge body 31 accommodates the liquid medicine LM. An end of the cartridge body 31 in the downward direction D is closed with the seal 32. The piston 33 is fitted in an end of the cartridge body 31 in the upward direction U. The seal 32 is broken by being pierced by the needle 2. If a force stronger than a predetermined strength is applied in the downward direction D after the seal 32 is broken, the piston 33 moves in the downward direction D relative to the cartridge body 31 and, thereby, pushes out the liquid medicine LM through the needle 2. That is, even if the force is applied in the downward direction D after the seal 32 is broken, the piston 33 moves being integrated with the cartridge body 31 without moving in the downward direction D relative to the cartridge body 31 if the cartridge body 31 is movable in the downward direction D against the reaction force of the spring 6.

The lower case 4 is substantially cylindrically configured as a lower casing of the administration device 1 by a pair of left and right members 4A and 4B being coupled with each other. Though the lower case 4 is configured with the pair of left and right members 4A and 4B being coupled with each other in the present embodiment, the lower case 4 is not limited thereto. The lower case 4 may be integrally configured by integral molding or the like. A lower end 45 (a first end) of the lower case 4 is open. In the lower case 4, the needle 2, the cartridge 3 and the like are housed in such a manner as to be movable in the downward direction D and the upward direction U. By the cartridge 3 to which the force in the downward direction D is applied being moved integrally with the needle 2 in the downward direction D, the needle 2 advances from the lower end 45 of the lower case 4, and it becomes possible to insert the needle 2 into the intradermal IN. Specifically, by the middle case 5 holding the cartridge 3 and the needle 2 being integrated, the cartridge 3 and the needle 2 are integrated.

On the inner surface of the lower case 4, a flange 41 to support the spring 6 is formed. On the inner surface of the lower case 4, on both sides in the left direction L and the right direction R in the downward direction D of the flange 41, paired recess portions 42 constituting the fixation release mechanism 8 are formed, respectively. On the lower end 45 of the lower case 4, a flange 43 that the needle base 7 that has moved in the downward direction D hits is formed in a ring shape on the inner surface side. The inner side of the ring-shaped flange 43 forms an opening 46 that vertically runs. The size and shape of the opening 46 are not restricted if it is possible to move the needle 2 through the opening 46. On the outer surface of the lower case 4, one ridge 44 is formed on each of both sides in the forward direction F and the backward direction B, the ridge 44 preventing falling off from the upper case 9 and enabling movement in the downward direction D and the upward direction U relative to the upper case 9. The ridges 44 vertically extend and are placed inside grooves 94 of the upper case 9, respectively.

The middle case 5 is substantially cylindrically configured. The cartridge 3 is housed in the middle case 5, and the middle case 5 integrated with the cartridge 3 is housed in the lower case 4. On the side surface of the middle case 5, paired windows 51 for visually confirming the liquid medicine LM accommodated in the cartridge 3 are formed on both sides in the left direction L and the right direction R, respectively. On the outer surface of the middle case 5, a flange 52 to support the spring 6 is formed in the upward direction U of the paired windows 51. On the outer surface of the middle case 5, on both sides in the left direction L and the right direction R in the downward direction D of the paired windows 51, paired holes 53 constituting the fixation release mechanism 8 are formed, respectively. On the downward direction D sides of the holes 53, recesses 56 are provided, respectively. The holes 53 vertically adjoin the recesses 56, respectively.

On the flange 52 of the middle case 5, paired sound boards 54 constituting the notification mechanism 20 are formed extending in the upward direction U, on both sides in the forward direction F and the backward direction B, respectively. On the sound board 54 on the forward direction F side, a claw 55 constituting the notification mechanism 20 is formed on the forward direction F side. On the sound board 54 on the backward direction B side, a claw 55 constituting the notification mechanism 20 is formed on the backward direction B side.

The spring 6 intervenes between the cartridge 3 and the case 4 so as to generate a reaction force and controls a pressing force of the case 4 against skin SK (see FIG. 5D and the like) when the cartridge 3 moves integrally with the needle 2 in the downward direction D. In response to a force applied to the cartridge 3 in the downward direction D being released, the spring 6 causes the cartridge 3 to move integrally with the needle 2 in the upward direction U. Specifically, the spring 6 intervenes between the flange 52 of the middle case 5 housing the cartridge 3 and the flange 41 of the lower case 4. The spring 6 expands/contracts as the middle case 5 storing the cartridge 3 moves relative to the lower case 4.

In the present invention, a pressing force control member (the spring 6) is only required to intervene between the cartridge 3 (a cartridge) and the lower case 4 (a case) in such a manner as to generate a reaction force directly or indirectly (via another member). In the present embodiment, the spring 6 intervenes between the cartridge 3 and the lower case 4 in such a manner as to indirectly generate a reaction force via the middle case 5.

As for the embodiment, it is described that the middle case 5 and the cartridge 3 are separate components, that is, the middle case 5 is not a part of the configuration of the cartridge 3. However, since the cartridge 3 and the middle case 5 are integrated, the middle case 5 can be considered to be a part of the configuration of the cartridge 3 from another point of view. Specifically, the cartridge 3 can be considered to have the cartridge body 31, the seal 32, the piston 33 and the middle case 5. When the cartridge 3 is considered as above, the pressing force control member (the spring 6) is thought to intervene between the cartridge 3 (the cartridge) and the lower case 4 (the case) so as to directly generate a reaction force.

The needle base 7 is housed in the lower case 4 and, in the initial state, supports the needle 2 at a position away from the cartridge 3. The needle base 7 is fixed to the lower case 4. By the fixation being released by the fixation release mechanism 8, it becomes possible for the needle base 7 to move integrally with the needle 2 in the downward direction D and the upward direction U. Specifically, the needle base 7 has a base body 71, paired guide pieces 72 and paired claws 73. The base body 71 is a plate extending in a direction orthogonal to the needle 2, causing the needle 2 to penetrate itself while supporting the needle 2. The paired guide pieces 72 are plates extending in the upward direction U from an edge of the base body 71 on both of the forward direction F and backward direction B sides, respectively, and guide movement of the needle base 7 relative to the lower case 4.

The paired claws 73 constitutes the fixation release mechanism 8. The paired claws 73 extend in the upward direction U from the edge of the base body 71 on both of the left direction L and right direction R sides, respectively, in such a manner as to become slightly closer to each other, and have a first claw head 73a and a second claw head 73b on ends in the upward direction U, respectively. Each of the paired first claw heads 73a has a shape protruding outside in the left and right direction. Each of the paired second claw heads 73b has a shape protruding inside in the left and right direction.

The fixation release mechanism 8 releases fixation of the needle base 7 to the lower case 4 after the cartridge 3 that has moved in the downward direction D come to be integrated with the needle 2. Specifically, the fixation release mechanism 8 is configured with the paired recess portions 42 of the lower case 4, the paired holes 53 of the middle case 5 and the paired claws 73 of the needle base 7. In the initial state, the paired claws 73 of the needle base 7 are bent outside by the paired second claw heads 73b being pushed outside by the recesses 56 of the middle case 5, respectively. The paired first claw heads 73a are fitted in the paired recess portions 42 of the lower case 4. Thereby, fixation of the needle base 7 to the lower case 4 is performed. The bending of the paired claws 73 of the needle base 7 to the outside is released by the middle case 5 moving in the downward direction D and the paired holes 53 of the middle case 5 reaching positions facing the paired second claw heads 73b. Then, the paired second claw heads 73b are fitted into the paired holes 53 of the middle case 5, and the paired first claw heads 73a get out of the paired recess portions 42 of the lower case 4. Thereby, the fixation of the needle base 7 to the lower case 4 is released.

The upper case 9 is substantially cylindrically configured as an upper casing of the administration device 1 by a pair of front and back members 9A and 9B being coupled with each other. Though the upper case 9 is configured by the pair of front and back members 9A and 9B being coupled with each other in the present embodiment, the upper case 9 is not limited thereto. The upper case 9 may be integrally configured by integral molding or the like. On the side surface of the upper case 9, paired windows 91 for visually confirming the liquid medicine LM accommodated in the cartridge 3 are formed on both sides in the left direction L and the right direction R, respectively. On the side surface of the upper case 9, on both sides in the forward direction F and the backward direction B, paired holes 92 constituting the notification mechanism 20 are formed, respectively.

On an upper end of the upper case 9, a top plate 93 is formed. In the top plate 93, a through hole 93a through which the pushing member 11 of the head cover 10 is inserted is formed. On the inner surface of the upper case 9, one groove 94 is formed on each of both sides in the forward direction F and the backward direction B. By being engage with the ridges 44 of the lower case 4, the grooves 94 prevents falling off from the lower case 4 and enables movement in the downward direction D and the upward direction U relative to the lower case 4.

The head cover 10 is a cover that covers the upper end of the upper case 9 and is fixed to the upper case 9. The pushing member 11 is provided inside the head cover 10. The pushing member 11 is a rod-shaped member that is inserted in the through hole 93a of the upper case 9 and has a length reaching the piston 33 of the cartridge 3. The pushing member 11 applies a force in the downward direction D to the cartridge 3 by moving in the downward direction D by being pushed by an operator.

The notification mechanism 20 generates a sound and a force to be transmitted to the operator by being engaged when administration of the liquid medicine LM is completed to thereby provide notification of the completion of the administration of the liquid medicine LM to the operator. Specifically, the notification mechanism 20 is configured with the paired sound boards 54 of the middle case 5, the paired claws 55 of the middle case 5 and the paired holes 92 of the upper case 9. In the initial state, the paired sound boards 54 of the middle case 5 are bent to the inside by the paired claws 55 being in contact with the inner surface of the upper case 9. By the middle case 5 moving in the upward direction U and the paired claws 55 of the middle case 5 reaching positions corresponding to the paired holes 92 of the upper case 9, the bending of the paired sound boards 54 of the middle case 5 to the inside is released and transformed to the outside at once. Then, the paired claws 55 enter the paired holes 92 of the upper case 9, and the sound boards 54 snap and hit the inner surface of the upper case 9. Thereby a sound and a force to be transmitted to the operator is generated.

The administration device 1 of the present embodiment is capable of inserting the needle 2 into a living body by, in a state of pressing the lower end 45 of the lower case 4 to the skin SK, causing the needle 2 to advance to the outside of the lower end 45 by causing the needle 2 to move to the lower end 45 side through the opening 46 of the lower end 45.

The administration device 1 is configured to, in the process of inserting the needle 2 into the living body by causing the needle 2 to move through the opening 46 of the lower end 45 of the lower case 4 so as to advance to the outside of the lower end 45, cause the spring 6 to continue generating a reaction force and to control a pressing force of the case 4 against the skin SK to be substantially constant so that the insertion depth of the needle 2 is substantially constant in a state in which the insertion of the needle 2 into the living body is completed. Since the pressing force changes according a pressing stroke, "causing the pressing force to be substantially constant" means not causing the pressing force to be substantially constant through the insertion process but causing the pressing force to be substantially constant irrespective of an operator. Further, the administration device 1 is configured to, in a process of discharging and administering the liquid medicine LM accommodated in the cartridge 3 through the needle 2 into the living body, cause the spring 6 to continue generating the reaction force so that the insertion depth of the needle 2 is substantially constant.

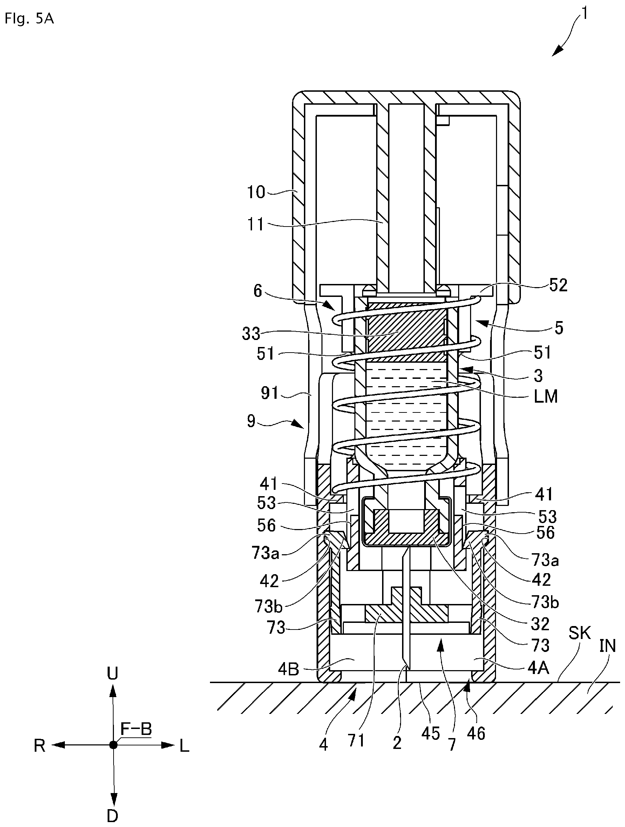

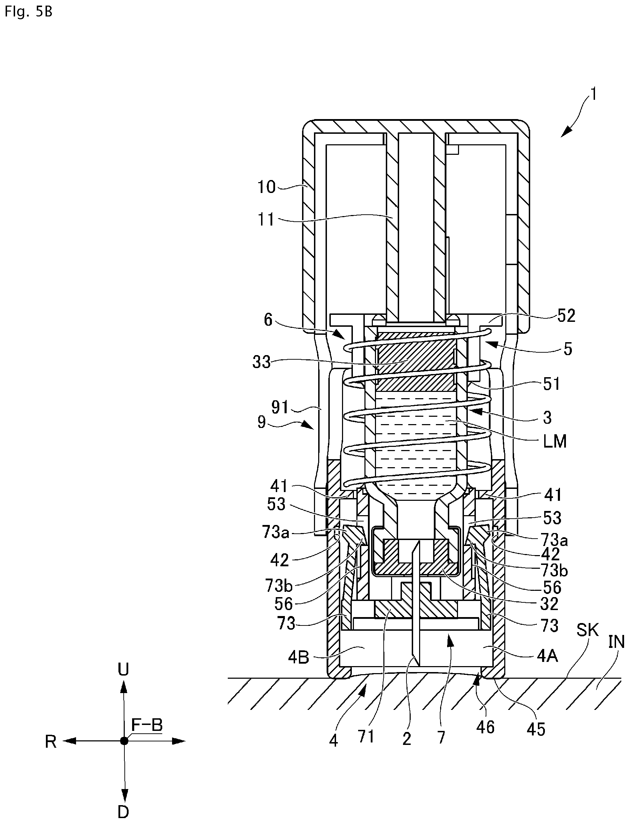

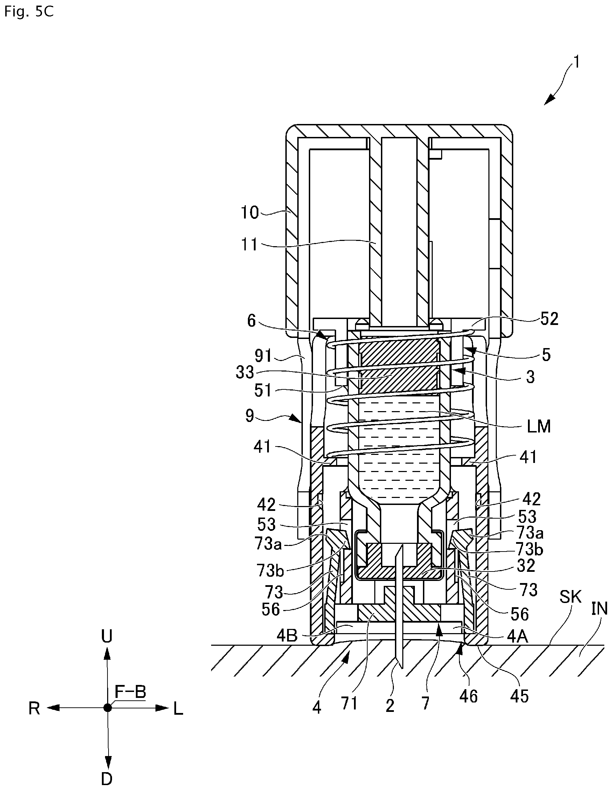

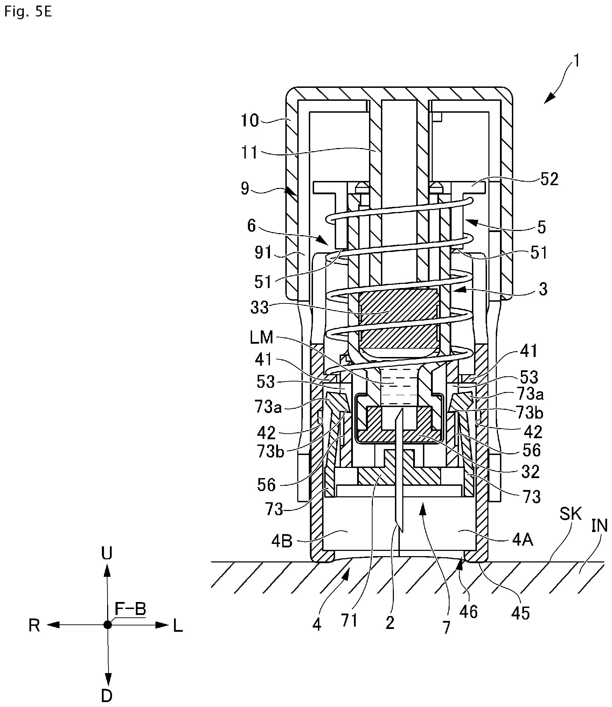

Next, an operation of the administration device 1 by the operator will be described using FIGS. 4A and 4B, and FIGS. 5A to 5E. FIG. 5A is a sectional perspective view of the administration device 1 shown in the direction of the arrow X-X in FIG. 1 and shows the initial state. FIG. 5B is a sectional view of the administration device 1 shown in the direction of the arrow X-X in FIG. 1 and shows the engaged state in which the cartridge 3 is integrated with the needle 2. FIG. 5C is a sectional view of the administration device 1 shown in the direction of the arrow X-X in FIG. 1 and shows a state at the time when administration of the liquid medicine LM is started. FIG. 5D is a sectional view of the administration device 1 shown in the direction of the arrow X-X in FIG. 1 and shows a state at the time when the administration of the liquid medicine LM is completed. FIG. 5E is a sectional view of the administration device 1 shown in the direction of the arrow X-X in FIG. 1 and shows a state in which the needle 2 is housed in the lower case 4 after administration of the liquid medicine LM is completed.

First, as shown in FIGS. 4A and 5A, in a state in which the first claw heads 73a of the claws 73 of the needle base 7 are fitted in the recess portions 42 of the lower case 4, and the needle base 7 (and the needle 2) is fixed to the lower case 4, the operator causes the lower end 45 of the lower case 4 to come into contact with the skin SK. After that, the operator pushes the head cover 10 in the downward direction D. Thereby, a force in the downward direction D is applied to the cartridge 3 via the pushing member 11, and, therefore, the force in the downward direction D is also applied to the middle case 5. Then, the middle case 5 moves in the downward direction D. As a result, the spring 6 intervening between the flange 52 of the middle case 5 and the flange 41 of the lower case 4 contracts (thereby, the reaction force of the spring 6 increases). Therefore, the lower case 4 moves in the downward direction D against the reaction force of the spring 6. The skin SK swells by being pushed by the lower end 45 of the lower case 4 that is open (the swelled state is shown in FIG. 5B) .

Then, as shown in FIGS. 4B and 5B, the needle 2 pierces the seal 32 of the cartridge 3. In the administration device 1 of the present embodiment, the timing when the needle 2 pierces the seal 32 is a constant timing irrespective of the operator because the timing is controlled by a push-in stroke (a push-in amount, a push-in depth) of the pushing member 11 of the head cover 10. The fixation release mechanism 8 releases fixation of the needle base 7 to the lower case 4. In the administration device 1 of the present embodiment, a fixation releasing operation by the fixation release mechanism 8 is controlled by the push-in stroke (the push-in amount, the push-in depth) of the pushing member 11 of the head cover 10. Specifically, the middle case 5 moves in the downward direction D accompanying pushing in by the pushing member 11 of the head cover 10, and, therefore, the positions of the second claw heads 73b of the claws 73 move from positions facing the recesses 56 of the middle case 5 to positions facing the holes 53 of the middle case 5. Then, the second claw heads 73b get into the holes 53. Thereby, the claws 73 transform to the inside, and the first claw heads 73a of the claws 73 get out of the recess portions 42 of the lower case 4. In this way, the fixation of the needle base 7 to the lower case 4 is released. Then, the needle 2 and the cartridge 3 become movable in the downward direction D being integrated with each other.

The needle 2 moves to the lower end 45 side through the opening 46 of the lower end 45 of the lower case 4, advances to the outside of the lower end 45 and is inserted into the living body. In such an insertion process, the spring 6 continues generating the reaction force, and the pressing force of the lower case 4 against the skin SK is controlled to be substantially constant. Thereby, in the state in which the insertion of the needle 2 into the living body is completed, the insertion depth of the needle 2 is substantially constant. The liquid medicine LM accommodated in the cartridge 3 is discharged through the needle 2 and administered into the living body. In such an administration process, the spring 6 continues generating the reaction force, and the insertion depth of the needle 2 is substantially constant.

After the administration of the liquid medicine LM, it becomes possible for the needle 2, the cartridge 3, the middle case 5 and the upper case 9 to move (retreat) in the upward direction U, being integrated with one another. If the fixation releasing operation by the fixation release mechanism 8 is controlled by the push-in force of the pushing member 11 of the head cover 10, there is a possibility that, due to variations of the push-in (operation) speed, the size of the member and the like, the timing of the fixation releasing by the fixation release mechanism 8 fluctuates, and, consequently, the pressing force against the skin SK also fluctuates.

As shown in FIG. 5C, when the operator further pushes the head cover 10 in the downward direction D, the needle 2 advances to the outside of the lower end 45 (in the downward direction D) and is inserted into the intradermal IN, and the needle base 7 comes into contact with the flange 43 of the lower case 4. Thereby, it becomes possible to administer the liquid medicine LM. Here, a frictional force between the cartridge 3 and the lower case 4 is smaller than a force required to move the piston 33 in the downward direction D against the liquid medicine LM accommodated (filled) in the cartridge 3. Therefore, the liquid medicine LM does not leak from the needle point of the needle 2 while the needle 2 and the cartridge 3 are moving being integrated with each other. After that, by the operator further pushing the head cover 10 in the downward direction D, the piston 33 moves in the downward direction D relative to the cartridge body 31, and the liquid medicine LM is pushed out through the needle 2.

As shown in FIG. 5D, when the operator further pushes the head cover 10 in the downward direction D, the administration of the liquid medicine LM is completed. Here, in the process of discharging the liquid medicine LM accommodated in the cartridge 3 through the needle 2 and administering the liquid medicine LM into the intradermal IN (until the administration of the liquid medicine LM is completed), it does not happen that the spring 6 intervening between the flange 52 of the middle case 5 and the flange 41 of the lower case 4 completely contracts (that parts of the coil linear member come into contact with one another, and the coil cannot transform). Therefore, the spring 6 continues generating the reaction force. The notification mechanism 20 provides notification of the completion of the administration of the liquid medicine LM to the operator. Specifically, by the claws 55 of the middle case 5 reaching the positions corresponding to the holes 92 of the upper case 9, the claws 55 get into the holes 92 (a state in which the movement of the cartridge 3 and the middle case 5 that are integrated with the needle 2 is prevented occurs, and, furthermore, a state in which the movement of the upper case 9 is prevented also occurs).

Due to such prevention of the movement, after the liquid medicine LM is administered, the needle 2, the cartridge 3, the middle case 5 and the upper case 9 are enabled to move (retreat) in the upward direction U being integrated with one another. Further, the sound boards 54 snap and hit the internal surface of the upper case 9, and a sound and a force to be transmitted to the operator are generated. Thereby, the operator can confirm the completion of the administration of the liquid medicine LM. After that, the operator stops the operation of pushing the head cover 10.

By the operator stopping the operation of pushing the head cover 10, the spring 6 expands and causes the lower case 4 to move to the first end 45 side as shown in FIG. 5E. Thereby, the needle 2, the cartridge 3, the middle case 5, the needle base 7, the upper case 9 and the head cover 10 move (retreat) in the upward direction U relative to the lower case 4 being integrated with one another. Thereby, the needle 2 is housed in the lower case 4.

According to the administration device 1 according to the present embodiment, for example, the following effects are obtained. The administration device 1 according to the present embodiment includes: the needle 2 to be inserted into a living body (the intradermal IN); the cartridge 3 capable of accommodating the liquid medicine LM to be administered into the living body through the needle 2; the case (the lower case 4) with the open or openable first end (the lower end 45) to be pressed against the skin SK, the needle 2 and the cartridge 3 being housed in such a manner as to be movable toward the lower end 45 side, and the needle 2 being capable of advancing to the outside of the first end 45 by being moved toward the first end 45 side; and the pressing force control member (the spring 6) intervening between the cartridge 3 and the case 4 so as to generate a reaction force and controlling a pressing force of the case 4 against the skin SK when the needle 2 moves toward the first end 45 side. In a process of discharging the liquid medicine LM accommodated in the cartridge 3 through the needle 2 to administer the liquid medicine LM into the living body (the intradermal IN), the spring 6 can continue generating the reaction force.

Therefore, according to the administration device 1 according to the present embodiment, the spring 6, which is the pressing force control member, intervenes between the cartridge 3 and the lower case 4 in such a manner as to generate a reaction force to control a pressing force of the lower case 4 against the skin SK. Moreover, in the process of discharging the liquid medicine LM accommodated in the cartridge 3 through the needle 2 and administering the liquid medicine LM into the living body (into the intradermal IN), the spring 6 can continue generating the reaction force. Therefore, it is possible to control the pressing force of the lower case 4 against the skin SK to be substantially constant, and, as a result, it is possible to easily cause the insertion depth of the needle 2 to be substantially constant.

In the process of administering the liquid medicine LM, since the spring 6 continues generating the reaction force, it is possible for the needle point of the needle 2 to, for example, even when the skin SK of a patient moves in a direction away from the administration device 1, automatically track the skin SK of the patient by contraction of the spring 6 being slightly relieved. In the present embodiment, the liquid medicine LM is administered in a state in which the needle base 7 and the lower case 4 are abutted to each other. Thus, it is because, when the skin SK of the patient moves in the direction away from the administration device 1, the lower case 4, the needle base 7 and the needle 2 track (follow) the skin SK of the patient by contraction of the spring 6 being relieved that the insertion depth becomes substantially constant by the reaction force by the spring 6 being continued.

If a needle is simply pressed against the skin SK, the skin SK is dented before the needle is inserted into the living body (into the intradermal IN), and, therefore, the insertion depth of the needle 2 is shallow. On the other hand, according to the administration device 1 according to the present embodiment, since the lower end 45 of the lower case 4 to be pressed against the skin SK is open, it is possible to swell the skin SK in a dome shape by the lower case 4 being pressed against the skin SK and then press the needle 2 against the skin SK. Thereby, it is possible to easily insert the needle 2 up to a position at a depth of about 1 to 2 mm. That is, according to the administration device 1, since a complicated operation like that of the Mantoux method is not required, even those who have not been specially trained can cause the insertion depth of the needle 2 to be substantially constant in a state in which insertion of the needle 2 into a living body is completed.

Conventionally, an administration device of such a type that an operator such as a nurse draws liquid medicine from a vial and then administers the liquid medicine has been mainly adopted. However, the work is complicated. Therefore, it is conceivable to adopt a prefilled-type administration device in which liquid medicine is accommodated in advance. In the case of the prefilled-type administration device, however, it is important to seal and store the liquid medicine to prevent deterioration. In comparison, according to the administration device 1 according to the present embodiment, since the prefilled-type cartridge 3 in which the liquid medicine LM is accommodated in advance is adopted, it is possible to protect the liquid medicine LM from oxidation, contamination and the like and keep a clean state.

In the present embodiment, the needle 2, the cartridge 3, the middle case 5 and the upper case 9 can move (retreat) in the upward direction U being integrated with one another after the liquid medicine LM is administered. In response to a force applied to the cartridge 3 toward the lower end 45 side being released, the spring 6 causes the cartridge 3 to move integrally with the needle 2 toward a side opposite to the lower end 45.

Thereby, in response to the force applied to the cartridge 3 toward the lower end 45 side (the downward direction D) being released after administration of the liquid medicine LM is completed, the spring 6 causes the needle 2 to move toward the side opposite to the lower end 45 side (the upward direction U), and the needle 2 is housed in the lower case 4. Therefore, it is possible to prevent an accident of inserting a used needle.

In the present embodiment, in a state in which movement of the cartridge 3 integrated with the needle 2 is prevented after administration of the liquid medicine LM is completed, the spring 6 causes the lower case 4 to move to the first end 45 side.

If movement of the cartridge 3 integrated with the needle 2 is not prevented after administration of the liquid medicine LM is completed, the needle 2 does not move into the lower case 4 unless the expansion force of the spring 6 is sufficiently strong, and the needle point of the needle 2 is not hidden in the lower case 4. In the present embodiment, in a state in which movement of the cartridge 3 integrated with the needle 2 is prevented after administration of the liquid medicine LM is completed, the spring 6 causes the lower case 4 to move to the first end 45 side. Therefore, even if the expansion force of the spring 6 is weak, the needle 2 can move into the lower case 4, and the needle point of the needle 2 can be hidden in the lower case 4 more certainly.

The administration device 1 according to the present embodiment includes the notification mechanism 20 that provides to the operator notification of completion of administration of the liquid medicine LM by generating a sound or a force to be transmitted to the operator by being engaged at the time of the completion of the administration of the liquid medicine LM.

Therefore, the operator can confirm the completion of the administration of the liquid medicine not visually but by a sound or a tactile sensation, and workability is high. Especially, whether the full amount of vaccine is administered or not is important in order to cause the liquid medicine to be sufficiently effective. According to the above, it is possible to prevent the operator from discontinuing administration though the full amount has not been administered.

The administration device 1 according to the present embodiment includes the needle base 7 supporting the needle 2 at a position away from the cartridge 3 and being in a state of fixation to the case 4 in the initial state and, in response to the fixation being released, becoming movable integrally with the needle 2 to the lower end 45 side; and the fixation release mechanism 8 releasing the fixation after the cartridge 3 that has moved to the lower end 45 side is integrated with the needle 2.

As examples of the prefilled-type administration device in which liquid medicine is accommodated in advance, for example, those such that a medicine chamber and a needle are integrated and such that a needle is attached to a medicine chamber when used are given. In the case of the type in which a medical chamber and a needle are integrated, since it is necessary to fit a cap to the needle point, increase in pain at the time of insertion accompanying deterioration of the needle point is a problem. In the case of the type in which a needle is attached to a medicine chamber when used, time and effort to attach a needle and a failure by an operator during the needle attaching work (a human error) are problems. In comparison, according to the administration device according to the present invention, the needle 2 and the cartridge 3 are automatically integrated, and administration of the liquid medicine LM is enabled. Therefore, the time and effort to attach the needle 2 is not required, and a failure by an operator does not occur.

The administration device 1 according to the present embodiment includes the pushing member 11 applying a force toward the lower end 45 side to the cartridge 3 by being pushed by the operator and moving to the lower end 45 side. The cartridge 3 includes the cartridge body 31 accommodating the liquid medicine LM, and the piston 33 pushing out the liquid medicine LM through the needle 2 by being pushed by the pushing member 11 and moving to the lower end 45 side relative to the cartridge body 31.

Therefore, the operator can perform a series of operations of integration of the needle 2 and the cartridge 3, insertion of the needle 2 into a living body (into the intradermal IN), administration of the liquid medicine LM and the like by one action of pushing the pushing member 11 to the lower end 45 side, and the operation is easy.

The present invention is not limited to the embodiment described above, and modifications, improvements and the like in a range in which the object of the present invention can be achieved are included in the present invention.

Though description has been made on a case where the administration device 1 is an intradermal syringe, the present invention is not limited thereto, and the administration device 1 may be a device for administration into subcutaneous tissue or muscle. In the case of the device for administration into subcutaneous tissue, a needle is inserted into subcutaneous tissue, and liquid medicine is administered to the subcutaneous tissue through the needle. In the case of the device for administration into muscle, a needle is inserted into muscle, and liquid medicine is administered into the muscle through the needle. The destination of administration of liquid medicine may be a living body other than intradermal, subcutaneous tissue and muscle.

The first end (the lower end 45) may be configured to be always open or may be configured to be openable only when used (a configuration of not being opened when not used).

In the embodiment described above, since the pushing member 11 that directly pushes the piston 33 of the cartridge 3 is provided, the series of operations of insertion of the needle 2 into a living body (into the intradermal IN), administration of the liquid medicine LM and the like can be performed by the one action of pushing the pushing member 11 to the lower end 45 side (via the head cover 10). The present invention is, however, is not limited thereto. For example, a configuration can be adopted in which the head cover 10 pushes the cartridge body 31 of the cartridge 3, and another pushing member (not shown) that is not integrated with the head cover 10 pushes the piston 33 of the cartridge 3. In this configuration, though the series of operations described before cannot be performed by one action but can be performed by a plurality of actions.

The pressing force control member is not limited to the spring 6 in a coil shape, and various kinds of elastic members and elastic structures are also possible. The pressing force control member is not limited to a completely elastic body if a reaction force is generated, and the one that has a damper property is also possible.

Though description has been made on a case where the notification mechanism 20 generates both a sound and a force to be transmitted to an operator as an example in the embodiment described above, the present invention is not limited thereto. The notification mechanism 20 may generate one of a sound and a force to be transmitted to an operator.

### EXPLANATION OF REFERENCE NUMERALS

- 1: administration device
- 2: needle
- 3: cartridge
- 31: cartridge body
- 32: seal
- 33: piston
- 4: lower case (case)
- 4A, 4B: member
- 41: flange
- 42: recess portion
- 43: flange
- 44: ridge
- 45: lower end (first end)
- 46: opening
- 5: middle case
- 51: window
- 52: flange
- 53: hole
- 54: sound board
- 55: claw
- 56: recess
- 6: spring (pressing force control member)
- 7: needle base
- 71: base body
- 72: guide piece
- 73: claw
- 73a: first claw head
- 73b: second claw head
- 8: fixation release mechanism
- 9: upper case
- 9A, 9B: member
- 91: window
- 92: hole
- 93: top plate
- 93a: through hole
- 94: groove
- 10: head cover
- 11: pushing member
- 20: notification mechanism
- IN: intradermal
- SK: skin
- F: forward direction
- B: backward direction
- L: left direction
- R: right direction
- D: downward direction (first end side)
- U: upward direction (side opposite to first end)

## Claims

1. An administration device comprising:
a needle to be inserted into a living body;
a cartridge capable of accommodating liquid medicine to be administered into the living body through the needle;
a case having an open or openable first end to be pressed against a skin, and housing therein the needle and the cartridge in such a manner that the needle and the cartridge are movable toward the first end side, the needle being capable of advancing to an outside of the first end by being moved toward the first end side; and
a pressing force control member intervening between the cartridge and the case so as to generate a reaction force and controlling a pressing force of the case against the skin when the needle moves toward the first end side,
the administration device being configured to insert the needle into the living body by, in a state in which the first end of the case is pressed onto the skin, causing the needle to move through an opening of the first end so as to advance the needle to the outside of the first end, wherein
in a process of inserting the needle into the living body by causing the needle to move through the opening of the first end so as to advance the needle to the outside of the first end, the administration device causes the pressing force control member to continue generating the reaction force and controls the pressing force of the case against the skin to be substantially constant so that insertion depth of the needle is substantially constant in a state in which insertion of the needle into the living body is completed, and
in a process of discharging and administering the liquid medicine in the cartridge through the needle into the living body, the administration device causes the pressing force control member to continue generating the reaction force so that the insertion depth of the needle is substantially constant.

2. The administration device according to claim 1, wherein
in response to the cartridge being released from a force applied toward the first end side, the pressing force control member causes the cartridge to move integrally with the needle toward a side opposite to the first end relative to the case.

3. The administration device according to claim 2, wherein
in a state in which movement of the cartridge integrated with the needle is prevented after completion of administration of the liquid medicine, the pressing force control member causes the cartridge to move integrally with the needle toward the side opposite to the first end side relative to the case.

4. The administration device according to claim 3, further comprising a notification mechanism configured to be engaged at a time of the completion of the administration of the liquid medicine to thereby provide notification of the completion of the administration of the liquid medicine to the operator.

5. The administration device according to any of claims 1 to 4, further comprising:
a needle base supporting the needle at a position away from the cartridge and being in a state of fixation to the case in an initial state and, becoming movable integrally with the needle toward the first end side upon being released from the fixation; and
a fixation release mechanism configured to release the fixation after the cartridge that has moved toward the first end side is integrated with the needle.

6. The administration device according to any of claims 1 to 5, further comprising a pushing member configured to apply a force toward the first end side to the cartridge by moving toward the first end side by being pushed by an operator,
wherein
the cartridge comprises:
a cartridge body accommodating the liquid medicine; and
a piston configured to push out the liquid medicine through the needle by being pushed by the pushing member and moving toward the first end side relative to the cartridge body.

7. The administration device according to any of claims 1 to 6, the administration device being for use for intradermal administration.
